# EUROPEAN PATENT APPLICATION

(11) **EP 1 295 577 A2**
(43) Date of publication of application: **26.03.2003**
(21) Application number: 02256474.4
(22) Date of filing: 18.09.2002
(51) Int. Cl.: A61F 2/46, A61F 2/44, A61B 5/103

(54) **Device for verifying the alignment of an endoprosthesis**

(30) Priority: 19.09.2001 US 955680
(71) Applicant: DePuy AcroMed, Inc., Raynham, MA 02767-0350 (US)
(72) Inventor: O'Neil, Michael J., West Barnstable, Massachusetts 02668 (US)
(74) Representative: Mercer, Christopher Paul

(57) **Abstract**

An alignment verification device (10) includes a spacer element (12) and an alignment guide surface (40). The spacer element (12) has proximal and distal portions and an insert engaging element (20) disposed on the distal portion. The proximal portion can be of such a design so as to serve as a handle for the verification device. The alignment guide surface (40) is affixed to the spacer element (12) and defines an alignment orifice (42). The alignment orifice (42) is spaced apart from the insert engaging element (20). A prosthesis (102) is also disclosed having an engaging element (50,70) and a visual indicator element (26). The engaging element (50,70) is configured to releasably engage the prosthesis engaging element (20) of the alignment verification device (10) so that, upon engagement, the alignment orifice (42) is spaced apart from the visual indicator element (26). The engagement of the alignment verification device (10) with the prosthesis (102) can thus be adapted to permit a sighting element (108) of an image obtaining device (106) to be aligned with the alignment orifice (42) and the visual indicator element (26) so that an image obtaining device (106) is aligned with the prosthesis (102) in a known orientation.

## Description

### FIELD OF INVENTION

The present invention pertains to a device and method for verifying the proper alignment of a surgically implanted device with an image obtaining device, such as an X-ray machine.

### BACKGROUND OF THE INVENTION

Advancing age, as well as injuries, can lead to changes in the various bones, discs, joints and ligaments of the body. In particular, these changes can manifest themselves in the form of damage or degeneration of an intervertebral disc, the result of which is mild to severe chronic back pain. Intervertebral discs serve as "shock" absorbers for the spinal column, absorbing pressure delivered to the spinal column. Additionally, they maintain the proper anatomical separation between two adjacent vertebra. This separation is necessary for allowing both the afferent and efferent nerves to exit and enter, respectively, the spinal column. However, these discs are susceptible to various different pathological processes. One example is the herniation of disc material into the nerve canal where it can impinge a spinal nerve and result in significant discomfort for the affected individual. Another example of a pathological process involves the hardening of the disc itself resulting in the loss of its ability to serve as a pressure absorber.

Treatment for a diseased or damaged disc can involve the removal of the affected disc. Once the affected disc has been removed, fusion of the opposing vertebra to one another can be surgically effected, or an artificial disc can be implanted so as to maintain spatial and functional physiological integrity of the spinal column.

Many spinal implants incorporate either lordotic or kyphotic angles in order to maintain the natural load bearing and kinematic characteristics of the patient's spine. A slight rotation of the implant about the local axis of the spine can cause misalignment of the desired angles and can significantly affect the ability to restore lordosis or kyphosis and the desired spinal load transfer and kinematics. During a surgical procedure to replace a diseased disc with an artificial one, the artificial disc is implanted by a surgeon and an image obtaining device, such as an X-ray machine, is brought into the operating room (typically mounted on a C-arm) in order to image the implanted prosthesis to determine whether it is properly placed within a patient.

A key step in obtaining images that can tell the surgeon whether the implant is properly aligned within the patient is to properly align the imaging device itself. Many times, this is done in the operating room simply by inserting the prosthetic disc, checking the angular orientation of the disc by visually determining whether an implant inserter tool connected to the disc is extending straight up from the operating table or is relatively aligned with respect to anatomical landmarks, moving the C-arm into position so that the X-ray or other imaging device is directly over the disc, and taking an X-ray or other image. If upon viewing the image, the imaging device is not properly aligned for making the needed determination, its position is adjusted and a new image is obtained. This process of aligning the imaging device may be required yet again if the implant is not correctly aligned and adjustment of the implant and further implant orientation verification is required. For well known reasons, it is preferable to minimize the patients exposure to x-rays and it would be desirable to take as few x-rays as possible during the procedure. In addition, it is also preferable to minimize the amount of time for the overall procedure, and thus reduce the patients exposure to anesthesia. Hence, it is desirable to minimize the time duration involved in the imaging verification step.

There exists a need to more efficiently verify the placement of an image obtaining device with respect to a surgical implant, especially a spinal disc implant, during the implantation procedure.

### SUMMARY OF THE INVENTION

The present invention relates to a device useful for implanting a prosthesis, such as an artificial intervertebral disc, within a patient in a safe and efficient manner. More particularly, the invention provides an alignment verification device that facilitates the correct alignment between an implanted prosthesis and an image obtaining device.

In one aspect of the instant invention, an alignment verification device comprising a spacer element and an alignment guide surface is provided. The spacer element has proximal and distal portions and an insert engaging element disposed on the distal portion. The proximal portion can be designed to serve as a handle for the alignment verification device. The alignment guide surface is affixed to the spacer element and defines an alignment orifice that is spaced apart from the insert engaging.

In particular embodiments, the spacer element can comprise two elongate members, each having an insert engaging element, and the insert engaging element or elements can be generally rectangular and sized to fit within a slot formed on the surface of a spinal disc insert prosthesis. The insert engaging element can further comprise a depth stop element.

In a further aspect, the invention provides a prosthesis alignment verification system including an alignment verification device as described above and a prosthesis. The prosthesis has an engaging element and a visual indicator element. The engaging element is configured to releasably engage the prosthesis engaging element of the alignment verification device so that, upon engagement, the alignment orifice is spaced apart from the visual indicator element. The engagement of the alignment verification device with the prosthesis can thus be adapted to permit a sighting element of an image obtaining device to be aligned with the alignment orifice and the visual indicator element so that an image obtaining device is aligned with the prosthesis in a known orientation.

Further embodiments of this aspect of the invention can also include a prosthesis inserter tool and/or an image obtaining device. The inserter tool has a prosthesis engaging element that conforms substantially in shape to the prosthesis engaging element of the alignment verification device. In this way, each prosthesis engaging element can engage the same engaging element on the prosthesis. An orientable image obtaining device for use in the invention can include a sighting element for aiding in orienting the image obtaining device. The sighting device can be aimable through the alignment orifice to the visual indicator element on the prosthesis to provide a visual indication that the image obtaining device is oriented in a predetermined orientation with respect to the prosthesis. In specific embodiments, the image obtaining device can be an X-ray imager provided on a C-arm, and the sighting device can be a laser pointer.

In a still further aspect, the invention provides a method for verifying the orientation of an image obtaining device with respect to an implanted prosthesis. Generally, the method includes providing an alignment verification device as described above, and engaging the alignment verification device to a prosthesis having an engagement element for releasable mating with the prosthesis engaging element on the alignment verification device. In this way, the alignment orifice is spaced apart from a visual indicator element disposed on the prosthesis resulting in a predetermined geometry between the visual indicator element and the alignment orifice. Next, an image obtaining device having a sighting element is aligned for imaging the implanted prosthesis by aligning the sighting element with the alignment orifice and the visual indicator element.

In a further embodiment of this aspect of the invention, an insertion tool is provided having prosthesis engaging elements that conform substantially in shape to the prosthesis engaging elements of the alignment verification device so that the insertion tool engages the same feature or features on the prosthesis that the alignment verification device engages. The prosthesis to be inserted is engaged to the insertion tool and implanted in a patient. The orientation of the prosthesis within the patient can be established as best as possible in the operating room during the insertion procedure using the insertion tool. The insertion tool is then disengaged from the prosthesis and the alignment verification device is engaged to the prosthesis employing the same engaging elements on the prosthesis from which the insertion tool was disengaged. Verification of the alignment of an image obtaining device can then proceed as described above.

### BRIEF DESCRIPTION OF THE DRAWINGS

The invention will be more fully understood from the following detailed description taken in conjunction with the accompanying drawings, in which:
FIG. 1 is a side view of an alignment verification device of the invention being used in surgery to verify the alignment of an imaging device with respect to an implanted prosthesis;
FIG. 2 is a side view of an alignment verification device of the invention interacting with a prosthetic device;
FIG. 3 is a perspective view of a spinal disc prosthesis which can form part of a system of the invention; and
FIG. 4 is perspective view of a spinal disc inserter and prosthesis which can form part of a system of the invention.

### DETAILED DESCRIPTION OF THE INVENTION

The present invention provides a device, a system, and a method useful for implanting a prosthesis within a patient in a safe and efficient manner. Although the invention is described primarily with reference to an intervertebral disc implant, it is understood that the alignment verification device can be used in conjunction with other types of implanted medical devices.

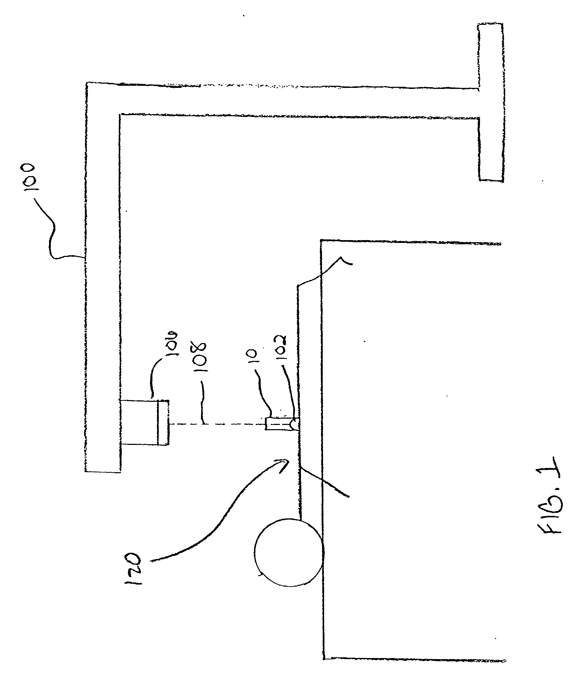

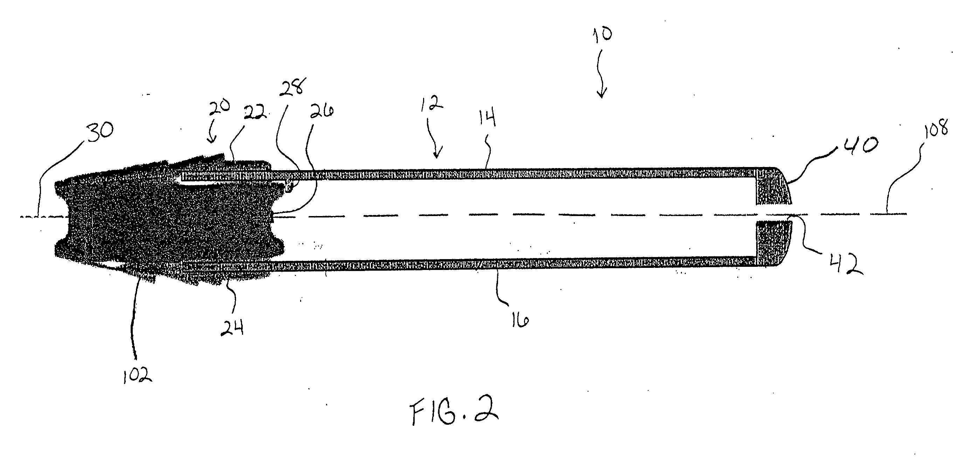

As shown in FIGS. 1 and 2, an alignment verification device 10 of the present invention is used to correctly align an image obtaining device 106 with a prosthetic device 102 placed surgically within a patient 120. Image obtaining device 106 is typically an X-ray image obtaining device, though other types of image obtaining devices could be used with the present invention. Image obtaining device 106 is generally mounted on a structure 100, such as a C-arm, to allow the image obtaining device to be moved around the operating room and to be aligned as desired. In addition, image obtaining device 106 typically includes a sighting element 108 such as a laser pointer for providing a visual indication as to the aiming or orientation of the image obtaining device.

Referring now to FIG. 2, alignment verification device 10 comprises a spacer element 12 having two elongate members 14, 16, and an alignment guide surface 40 defining an alignment orifice 42. The spacer element 12 has an insert engaging element 20 on its distal end which includes individual prosthesis engaging elements 22, 24 on a distal end of each of elongate members 14, 16, respectively. Insert engaging element 20 interacts with prosthesis 102 to place alignment orifice 42 into a predetermined geometric relationship with, and spaced apart from, the prosthesis so that sighting element 108 can be aimed through alignment orifice 42 to strike a predetermined visual indicator point 26 to provide a visual indication that image obtaining device 106 (FIG. 1) has been placed in a known orientation with respect to prosthesis 102, allowing verification of the orientation of the prosthesis to proceed efficiently.

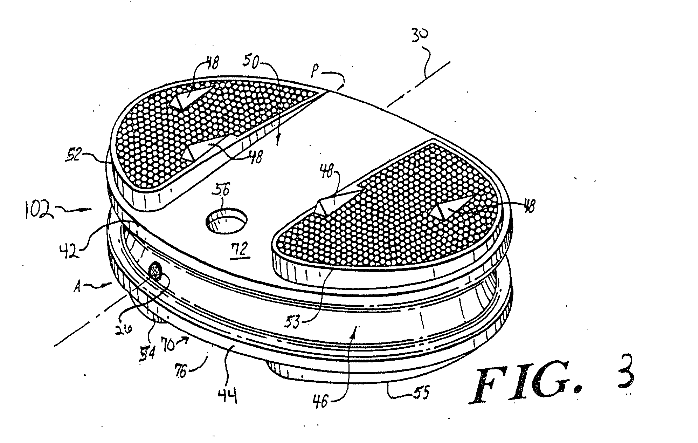

A person of ordinary skill in the art will recognize that other alignment verification device 10 configurations could provide the necessary predetermined geometric relationship between prosthesis 102 and alignment orifice 42. For example, spacer element 12 could have only one elongate member 14, or more than two. Similarly, insert engaging element 20 could consist of a single individual insert engaging element 22, or could include any manner of engagement between prosthesis 102 and spacer element 12 that would result in a predetermined geometry between prosthesis 102 and alignment orifice 42. In the illustrated embodiment, insert engaging element 20 comprises generally rectangular individual prosthesis engaging elements 22, 24, each shaped to fit in slots (FIG. 3) formed on inferior and superior surfaces of prosthesis 102. Insert engaging element 20 can also include depth stop 28. In other embodiments, insert engaging element 20 could be a threaded element configured to mate with a correspondingly threaded element on the insert, or one or more pin elements provided on one or the other of spacer element 12 and prosthesis 102 with a corresponding recess or recesses provided on the other. Where such a pin and recess configuration is employed with only one pin and one recess, it may be preferable to shape the pin and recess so that they resist rotation between alignment device 10 and prosthesis 102 in order to provide the previously mentioned predetermined geometry.

In a preferred embodiment, the predetermined geometry places alignment orifice 42 in a spaced apart relationship with respect to visual indicator point 26 on prosthesis 102 along a transverse axis 30 defined by the intersection of transverse and sagittal planes passing through the prosthesis and including the visual indicator point. In this way, the distance of the spacing between visual indicator 26 and alignment orifice 42 is not critical to achieving a predetermined geometry and determining correct orientation of image obtaining device 106.

An exemplary prosthesis 102 useful with the invention is illustrated in further detail in FIG. 3. Prosthesis 102 has a first end plate 42 and a second end plate 44. The first end plate 42 has a bone-facing surface 72 and an opposed mating surface, and the second end plate 44 has a bone-facing surface 76 and an opposed mating surface. An elastomeric core 46 is interposed between and attached to the mating surfaces of the first and second end plates 42, 44.

Illustrated bone facing surface 72 of the first end plate 42 includes adjacent lobes 52, 53 separated by slot 50. Bone-facing surface 76 of the second end plate 44 can similarly include lobes 54, 55 separated by slot 70. Either or both of the bone-facing surfaces 72, 76 of the first end plate 42 and the second end plate 44 can include one or more bone-penetrating, wedge-like fins 48 protruding therefrom, in particular these wedge-like fins can be formed on lobes 52, 53, 54, 55.

Slots 50, 70 form an alignment verification engaging element. That is, generally rectangular individual prosthesis enlargements 22, 24 (FIG. 2) fit snugly enough in generally rectangular slots 50, 70 to locate the alignment orifice 42 (FIG. 2) at the desired predetermined geometry spaced apart from the indicator element 26. Slots, 50, 70 may also include one or more extraction recesses 56, formed as blind bores in the slot surface. In addition, a further engaging element may be provided on prosthesis engaging element 20 to engage recess 56 to provide further positive engagement between prosthesis 102 and alignment verification device 10.

In the illustrated embodiment, the dimensions and geometry of the end plates 42, 44 and the lobes 52, 53, 54, 55 cause the overall thickness of prosthesis 102 to taper from the anterior face (A) to the posterior face (P) at an angle in the range of about 2° to 35°, and more preferably about 5° to 15°. Thus, the thickness of prosthesis 102 is greatest when measured between the anterior portions of the lobes, and least when measured between the posterior portions of the lobes. The thickness T of prosthesis 102 at the anterior side is in the range of about 5 to 21 mm, while the thickness T at the posterior side is in the range of about 1 mm to 15 mm. Other known artificial disc prostheses that can be employed within the spirit of the invention include those disclosed in United States Patent Nos. 5,683,465 to Shinn et al.; 5,674,294 to Bainville et al.; 5,458,643 to Oka et al.; 5,306,309 to Wagner et al.; and 4,759,769 to Hedman, et al., each of which is hereby incorporated by reference.

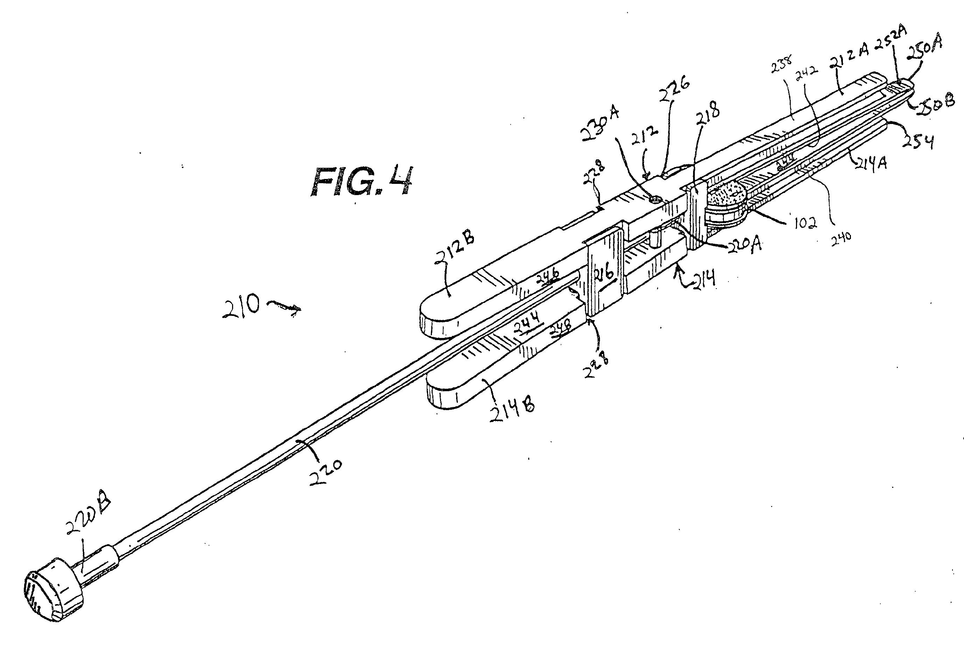

The invention can also be provided as a system for installing and verifying the placement orientation of a prosthesis including a prosthesis, a prosthesis installation tool, and an alignment verification device. Exemplary prosthesis and alignment verification devices have been described above, and an exemplary prosthesis insertion tool 210 is now described by reference to FIG. 4. Installation tool 210 comprises opposed levers 212, 214 each having a distal portion 212A, 214A and a proximal, handle portion 212B, 214B. Disposed between the levers 212, 214 is a fulcrum 216 and pusher block 218, which is disposed distally of the fulcrum 216. The pusher block 218 is selectively movable from an initial position distal of the fulcrum 216 to a final location adjacent a distal end of the levers. A pusher rod 220, which facilitates selective movement of the pusher block, has a distal end 220A connected to the pusher block and proximal handle end 220B.

A prosthesis, such as an artificial disk 102, is positioned between the levers, distal to the pusher block 18. Preferably the levers 212 and 214 are elongate elements that are mirror images of each other and the proximal portion 212B, 214B of each lever may include an indented region 228 for receiving the fulcrum 216. The proximal region of each lever 212B, 214B may also include a bore 230A, 230B which is adapted to seat a bolt 232 that enables control of the spacing between levers so that the pusher block accurately engages prosthesis 102.

The distal portion of each lever 212A, 214A features outwardly facing surfaces 238, 240 (illustrated as top and bottom surfaces, respectively) and inwardly facing surfaces 242, 244 upon which prosthesis 102 rides during an installation procedure. Distal portions 212A, 214A of levers 212, 214 also have blade tips 250A, 250B formed at the distal ends of the levers. The blade tips are sized and configured to facilitate their placement between vertebral bodies to aid in placement of prosthesis 102 between the vertebral bodies.

The engagement between inner surfaces 242, 244 of levers 212, 214 (which function as prosthesis engaging elements) and slots 50, 70 (FIG. 3) of prosthesis 102 allows the prosthesis to slide into place between vertebral bodies in response to movement of pusher block 218, while maintaining the rotational orientation of prosthesis 102. In order to a achieve this result, levers 212, 214, particularly in the area of inner surfaces 242, 244, can be generally rectangularly shaped and sized to fit within slots 50, 70 (FIG. 3) of prosthesis 102. Conveniently, prosthesis engaging elements 22, 24 of alignment verification device 10 can conform substantially in shape to the prosthesis engaging elements of insertion tool 210, allowing both alignment verification device 10 and insertion tool 210 to engage the same features on prosthesis. In one aspect, the invention includes both an insertion tool and an alignment verification device having substantially conforming prosthesis engaging elements. In another aspect, a system having such an insertion tool and alignment verification device further includes a prosthesis having an engagement feature that is engageable by the prosthesis engaging element on each tool.

A person of ordinary skill in the art will appreciate that the size and shape of the levers may vary. Generally, however, the overall length of the levers is about 200 to 400 mm, with proximal portion 212B, 214B (proximal end to shoulder 226) having a length of about 100 to 300 mm and the distal portion 212A, 214A (shoulder 226 to blade tips) having a length of about 100 to 300 mm.

The instant invention also includes a method for verifying the orientation of an image obtaining device with respect to an implanted prosthesis. Generally, the method includes providing an alignment verification device comprising a spacer element having proximal and distal portions with a prosthesis engaging element on its distal portion and an alignment guide surface. The alignment guide surface is affixed to the spacer element and defines an alignment orifice, the alignment orifice being spaced apart from the insert engaging element in a predetermined geometry. The alignment verification device is then engaged to a prosthesis having an engagement element for releasable mating with the prosthesis engaging element on the alignment verification device so that the alignment orifice is spaced apart from a visual indicator element disposed on the prosthesis resulting in a predetermined geometry between the visual indicator element and the alignment orifice. Next, an image obtaining device having a sighting element is aligned for imaging the implanted prosthesis by aligning the sighting element with the alignment orifice and the visual indicator element. An image of the implanted prosthesis can then be obtained with the assurance that the image obtaining device has the desired orientation with respect to the prosthesis. The correct orientation of the prosthesis with respect to the patent can then be determined.

A method of the invention can further include the provision of an insertion tool having prosthesis engaging elements that conform substantially in shape to the prosthesis engaging elements of the alignment verification device so that the insertion tool engages the same feature or features on the prosthesis that the alignment verification device engages. The prosthesis to be inserted is engaged to the insertion tool and implanted in a patient. The orientation of the prosthesis within the patient can be established as best as possible in the operating room during the insertion procedure by visually aligning the insertion tool with respect to anatomical features of the patient. The insertion tool is then disengaged from the prosthesis and the alignment verification device is engaged to the prosthesis employing the same engaging elements on the prosthesis from which the insertion tool was disengaged. Imaging can then proceed as described above.

Once the image is obtained as described above, a doctor can use the image to determine whether the prosthesis is correctly aligned within the patent knowing that the orientation of the prosthesis within the image is as desired. Thus, a single image can be taken to verify the orientation of the implant, rather than possibly obtaining multiple images simply to align the image obtaining device with the prosthesis before moving on to verify the orientation of the prosthesis with respect to the patient. If the orientation of the prosthesis within the patient is as desired, the surgical procedure can be competed. If the orientation is not as desired, the orientation of the prosthesis is readjusted, typically by re-engaging either the insertion tool or the alignment verification tool to the prosthesis and manually adjusting its orientation, then employing the alignment verification device again to align the image obtaining device with the prosthesis and obtaining an image. This process can be repeated until the desired orientation of the prosthesis within the patient is achieved.

One of ordinary skill in the art will appreciate further features and advantages of the invention based on the above-described embodiments. Accordingly, the invention is not to be limited by what has been particularly shown and described, except as indicated by the appended claims.

## Claims

1. An alignment verification device, comprising:
a spacer element having proximal and distal portions and an insert engaging element disposed on the distal portion; and
an alignment guide surface affixed to the spacer element and defining an alignment orifice, the alignment orifice being spaced apart from the insert engaging element.

2. The device of claim 1, wherein the spacer element includes two elongate members with the alignment guide fixed between the elongate members, each elongate member having an insert engaging element.

3. The device of claim 1 or claim 2, wherein the insert engaging element or each insert engaging element is a generally rectangular element sized to fit within a slot formed on the surface of a spinal disc insert prosthesis.

4. The device of claim 3, wherein the insert engaging element or at least one of the insert engaging elements includes a depth stop element.

5. A prosthesis alignment verification system, comprising:
an alignment verification device including
a spacer element having proximal and distal portions and a prosthesis engaging element disposed on the distal portion; and
an alignment guide surface affixed to the spacer element and defining an alignment orifice, the alignment orifice being spaced apart from the prosthesis engaging element; and
a prosthesis having an engaging element and a visual indicator element, the engaging element being configured to releasably engage the prosthesis engaging element of the alignment verification device so that, upon engagement, the alignment orifice is spaced apart from the visual indicator element.

6. The system of claim 5, wherein the engagement of the alignment vertification device with the prosthesis is adpated to permit a sighting element of an image obtaining device to be aligned with the alignment orifice and the visual indicator element so that an image obtaining device is aligned with the prosthesis in a known orientation.

7. The system of claim 6, wherein the prosthesis engaging element is generally rectangularly shaped and the engaging element of the prosthesis is a slot configured to engage the prosthesis engaging element.

8. The system of claim 6, wherein the spacer element includes two elongate members, each having a prosthesis engaging element, and the prosthesis includes two engaging elements, each configured to engage one of the prosthesis engaging elements.

9. The system of claim 7 or claim 8, wherein the prosthesis engaging element or at least one of the prosthesis engaging elements includes a depth stop element.

10. The system of claim 7, wherein the prosthesis is a spinal disc prosthesis.

11. The system of claim 10, wherein the spinal disc prosthesis incorporates an angle.

12. The system of claim 10, wherein the spinal disc prosthesis includes at least one bone facing surface having a slot as the engaging element, the prosthesis engaging element being sized to engage the slot.

13. The system of claim 12, wherein the spinal disc prosthesis includes two opposed bone facing surfaces each having a slot as the engaging element, the spacer element comprising two elongate members each having a prosthesis engaging element sized to engage a slot on the spinal disc prosthesis.

14. The system of claim 6, further comprising a prosthesis inserter tool, the inserter tool having a prosthesis engaging element conforming substantially in shape to the prosthesis engaging element of the alignment verification device so that each prosthesis engaging element can engage the same engaging element on the prosthesis.

15. The system of claim 6, further comprising an orientable image obtaining device including a sighting element for aiding in orienting the image obtaining device, the sighting device being aimable through the alignment orifice to the visual indicator element to provide a visual indication that the image obtaining device is oriented in a predetermined orientation with respect to the prosthesis.

16. The system of claim 15, wherein the sighting element is a laser pointer.

17. A method for verifying the orientation of an image obtaining device with respect to an implanted prosthesis, comprising the steps of:
providing an alignment verification device including
a spacer element having proximal and distal portions and a prosthesis engaging element disposed on the distal portion; and
an alignment guide surface affixed to the spacer element and defining an alignment orifice, the alignment orifice being spaced apart from the prosthesis engaging element;
engaging the alignment verification device to the implanted prosthesis, the prosthesis having an engaging element and a visual indicator element, the engaging element configured to releasably engage the prosthesis engaging element of the alignment verification device so that, upon engagement, the alignment orifice is spaced apart from the visual indicator element;
orienting the image obtaining device so that a sighting element on the image obtaining device is aimed through the alignment orifice to the visual indicator element to provide a visual indication that a predetermined orientation between the image obtaining device and the prosthesis has been achieved.

18. The method of claim 17, further comprising the following steps before engagement of the alignment verification device to the implanted prosthesis:
providing an insertion tool having a prosthesis engaging element that conforms substantially in shape to the prosthesis engaging element of the alignment verification device so that the insertion tool engages the same engaging element on the prosthesis that the alignment verification device engages;
engaging the prosthesis to be inserted to the insertion tool;
implanting the prosthesis in a patient in a desired location; and
disengaging the insertion tool from the prosthesis.

19. The method of claim 18, wherein the alignment verification device is engaged to the prosthesis employing the same engaging element on the prosthesis from which the insertion tool was disengaged.
